# EUROPEAN PATENT SPECIFICATION

(11) **EP 1 590 484 B1**
(45) Date of publication and mention of the grant of the patent: **19.08.2009**
(21) Application number: 04706664.2
(22) Date of filing: 30.01.2004
(51) Int. Cl.: C12Q 1/68

(54) **METHOD FOR THE DETECTION OF CYTOSINE METHYLATION PATTERNS WITH HIGH SENSITIVITY**
VERFAHREN ZUM NACHWEIS VON CYTOSINMETHYLIERUNGSMUSTERN MIT HOHER EMPFINDLICHKEIT
TECHNIQUE DE DETECTION DE CONFIGURATIONS DE METHYLATION DE CYTOSINE A HAUTE SENSIBILITE

(30) Priority: 30.01.2003 DE 10304219
(43) Date of publication of application: 02.11.2005
(73) Proprietor: Epigenomics AG, 10178 Berlin (DE)
(72) Inventor: BERLIN, Kurt, 14532 Stahnsdorf (DE)
(74) Representative: Schubert, Klemens
(86) International application number: PCT/EP2004/000856
(87) International publication number: WO 2004/067775

(56) References cited:
- WO-A-00/46398
- WO-A-00/70090
- WO-A-01/44504
- WO-A-01/76451
- WO-A-97/37041
- WO-A-99/60007
- US-A- 5 866 336
- US-B1- 6 265 171
- OLEK ALEXANDER ET AL: "A modified and improved method for bisulphite based cytosine methylation analysis" NUCLEIC ACIDS RESEARCH, vol. 24, no. 24, 1996, pages 5064-5066, XP002106408 ISSN: 0305-1048 cited in the application
- WORM JESPER ET AL: "In-tube DNA methylation profiling by fluorescence melting curve analysis" CLINICAL CHEMISTRY, vol. 47, no. 7, July 2001 (2001-07), pages 1183-1189, XP002298308 ISSN: 0009-9147
- OLEJNIK J ET AL: "Photocleavable peptide-DNA conjugates: synthesis and applications to DNA analysis using MALDI-MS" NUCLEIC ACIDS RESEARCH, OXFORD UNIVERSITY PRESS, SURREY, GB, vol. 27, no. 23, 1 December 1999 (1999-12-01), pages 4626-4631, XP002220753 ISSN: 0305-1048
- YU ET AL: "SPECIFIC INHIBITION OF PCR BY NON-EXTENDABLE OLIGONUCLEOTIDES USING A 5' TO 3' EXONUCLEASE-DEFICIENT DNA POLYMERASE" BIOTECHNIQUES, EATON PUBLISHING, NATICK, US, vol. 23, no. 4, October 1997 (1997-10), pages 714,716-720, XP001041499 ISSN: 0736-6205
- AUER T ET AL: "SELECTIVE AMPLIFICATION OF RNA UTILIZING THE NUCLEOTIDE ANALOG DITPAND THERMUS THERMOPHILUS DNA POLYMERASE" NUCLEIC ACIDS RESEARCH, OXFORD UNIVERSITY PRESS, SURREY, GB, vol. 24, no. 24, 1996, pages 5021-5025, XP002916438 ISSN: 0305-1048

## Description

The present invention concerns a method for the detection of cytosine methylation in DNA samples.

The levels of observation that have been well studied in molecular biology according to developments in methods in recent years include the genes themselves, the transcription of these genes into RNA and the translation to proteins therefrom. Which gene is turned on during the course of development of an individual, and how activation and inhibition of certain genes in certain cells and tissues is controlled can be correlated with the extent and nature of the methylation of genes or of the genome. In this regard, pathogenic states are reflected in a modified methylation pattern of individual genes or of the genome.

5-Methylcytosine is the most frequent covalently modified base in the DNA of eukaryotic cells. For example, it plays a role, in the regulation of transcription, in genetic imprinting and in tumorigenesis. The identification of 5-methylcytosine as a component of genetic information is thus of considerable interest. 5-Methylcytosine positions, however, cannot be identified by sequencing, since 5-methylcytosine has the same base-pairing behavior as cytosine. In addition, in the case of a PCR amplification, the epigenetic information which is borne by the 5-methylcytosines is completely lost.

A relatively new method that in the meantime has become the most widely used method for investigating DNA for 5-methylcytosine is based on the specific reaction of bisulfite with cytosine, which, after subsequent alkaline hydrolysis, is then converted to uracil, which corresponds in its base-pairing behavior to thymine. In contrast, 5-methylcytosine is not modified under these conditions. Thus, the original DNA is converted so that methylcytosine, which originally cannot be distinguished from cytosine by its hybridization behavior, can now be detected by "standard" molecular biology techniques as the only remaining cytosine, for example, by amplification and hybridization or sequencing. All of these techniques are based on base pairing, which is now fully utilized. The prior art concerning sensitivity is defined by a method for bisulfite treatment that incorporates the DNA to be investigated in an agarose matrix, so that the diffusion and renaturation of the DNA are prevented (bisulfite reacts only on single-stranded DNA) and all precipitation and purification steps are replaced by rapid dialysis (Olek A, Oswald J, Walter J. A modified and improved method for bisulphite based cytosine methylation analysis. Nucleic Acids Res. 1996 Dec 15;24(24):5064-6). By this method the DNA of individual cells can be treated, which illustrates the potential of the method. However, up until now, only individual regions of up to approximately 3000 base pairs long have been treated; a global treatment of cells for thousands of possible regions is not possible. In addition, also this method cannot reliably convert very small fragments of small quantities of sample. These are lost despite the protection from diffusion through the matrix.

An overview of other known possibilities for detecting 5-methylcytosines can be derived from the following review article: Rein T, DePamphilis ML, Zorbas H. Identifying 5-methylcytosine and related modifications in DNA genomes. Nucleic Acids Res. 1998 May 15;26(10):2255-64.

The bisulfite technique has previously been applied in research only, with a few exceptions (e.g., Zeschnigk M, Lich C, Buiting K, Dörfler W, Horsthemke B. A single-tube PCR test for the diagnosis of Angelman and Prader-Willi syndrome based an allelic methylation differences at the SNRPN locus. Eur J Hum Genet 1997 Mar-Apr;5(2):94-8). However, in any case short, specific segments of a known gene are amplified after a bisulfite treatment and are either completely sequenced (Olek A, Walter J. The pre-implantation ontogeny of the H19 methylation imprint. Nat Genet. 1997 Nov.;17(3):275-6) or individual cytosine positions are detected by a "primer extension reaction" (Gonzalgo ML, Jones PA. Rapid quantitation of methylation differences at specific sites using methylation-sensitive single nucleotide primer extension (Ms-SNuPE). Nucleic Acids Res. 1997 Jun. 15;25(12):2529-31, WO 95/00669) or an enzyme step (Xiong Z, Laird PW. COBRA: a sensitive and quantitative DNA methylation assay. Nucleic Acids Res. 1997 Jun. 15;25(12):2532-4). Detection by hybridization has also been described (Olek et al., WO 99/28498).

Urea improves the efficiency of bisulfite treatment prior to sequencing of 5-methylcytosine in genomic DNA (Paulin R, Grigg GW, Davey MW, Piper AA. Urea improves efficiency of bisulphite-mediated sequencing of 5-methylcytosine in genomic DNA. Nucleic Acids Res. 1998 Nov 1;26(21):5009-10).

Other publications concerning the application of the bisulfite technique for the detection of methylation in individual genes are: Grigg G, Clark S. Sequencing 5-methylcytosine residues in genomic DNA. Bioassays. 1994 Jun.;16(6):431-6, 431; Zeschnigk M, Schmitz B, Dittrich B, Buiting K, Horsthemke B, Dörfler W. Imprinted segments in the human genome: different DNA methylation patterns in the Prader-Willi/Angelman syndrome region as determined by the genomic sequencing method. Hum Mol Genet. 1997 Mar;6(3):387-95; Feil R, Charlton J, Bird AP, Walter J, Reik W. Methylation analysis on individual chromosomes: improved protocol for bisulfite genomic sequencing. Nucleic Acids Res. 1994 Feb. 25;22(4):695-6; Martin V, Ribieras S, Song-Wang X, Rio MC, Dante R. Genomic sequencing indicates a correlation between DNA hypomethylation in the 5' region of the pS2 gene and in its expression in human breast cancer cell lines. Gene. 1995 May 19;157(1-2):261-4; WO 97/46705, WO 95/15373 and WO 97/45560.

Another known method is the so-called methylation-sensitive PCR (Herman JG, Graff JR, Myohanen S, Nelkin BD, Baylin SB (1996), Methylation-specific PCR: a novel PCR assay for methylation status of CpG islands. Proc Natl Acad Sci U S A. Sep 3;93(18):9821-6). For this method, primers are used which hybridize either only to a sequence that is generated by the bisulfite treatment of a DNA which is unmethylated at the respective position, or, vice versa, primers which bind only to a nucleic acid which is generated by the bisulfite treatment of a DNA which is methylated at the respective position. Amplificates can be produced accordingly with these primers, the detection of which in turn supplies indications of the presence of a methylated or unmethylated position in the sample to which the primers bind.

A newer method is also the detection of cytosine methylation by means of a Taqman PCR, which has become known as MethyLight (WO 00/70090). It is possible with this method to detect the methylation state of individual positions or a few positions directly in the course of the PCR, so that a subsequent analysis of the products becomes superfluous.

An overview of the prior art in oligomer array production can also be derived from a special issue of Nature Genetics which appeared in January 1999 (Nature Genetics Supplement, Volume 21, January 1999), the literature cited therein and from US Patent 5,994,065 on methods for the production of solid supports for target molecules such as oligonucleotides in the case of reduced nonspecific background signal.

Probes with multiple fluorescent labels have been used for scanning an immobilized DNA array. Particularly suitable for fluorescent labels is the simple introduction of Cy3 and Cy5 dyes at the 5'-OH of the respective probe. The fluorescence of the hybridized probes is detected, for example, by means of a confocal microscope. Among many others, the dyes Cy3 and Cy5 are commercially available.

Matrix-assisted laser desorptions/ionization mass spectrometry (MALDI-TOF) is a very powerful development for the analysis of biomolecules (Karas M, Hillenkamp F. Laser desorption ionization of proteins with molecular masses exceeding 10,000 daltons. Anal Chem. 1988 Oct. 15;60(20):2299-301). An analyte is embedded in a light-absorbing matrix. The matrix is vaporized by a short laser pulse and the analyte molecule is transported unfragmented into the gaseous phase. The analyte is ionized by collisions with matrix molecules. An applied voltage accelerates the ions in a field-free flight tube. Ions are accelerated to varying degrees based on their different masses. Smaller ions reach the detector sooner than larger ions.

MALDI-TOF spectroscopy is excellently suitable for the analysis of peptides and proteins. The analysis of nucleic acids is somewhat more difficult (Gut, I. G. and Beck, S. (1995), DNA and Matrix Assisted Laser Desorption Ionization Mass Spectrometry. Molecular Biology: Current Innovations and Future Trends 1: 147-157). For nucleic acids, the sensitivity is approximately 100 times poorer than for peptides and decreases overproportionally with increasing fragment size. For nucleic acids, which have a backbone with a multiple negative charge, the ionization process via the matrix is basically inefficient. In MALDI-TOF spectroscopy, the choice of matrix plays an imminently important role. Several very powerful matrices, which produce a very fine crystallization, have been found for the desorption of peptides. In the meantime, several effective matrices have been developed for DNA, but the difference in sensitivity has not been reduced thereby. The difference in sensitivity can be reduced by modifying the DNA chemically in such a way that it resembles a peptide.

Phosphorothioate nucleic acids, in which the usual phosphates of the backbone are substituted by thiophosphates, can be converted by simple alkylation chemistry into a charge-neutral DNA (Gut, I. G. and Beck, S. (1995), A procedure for selective DNA alkylation and detection by mass spectrometry. Nucleic Acids Res. 23: 1367-1373). The coupling of a "charge tag" to this modified DNA results in an increase in sensitivity to the same amount as is found for peptides. Another advantage of charge tagging is the increased stability of the analysis in the presence of impurities, which make the detection of unmodified substrates very difficult.

Genomic DNA is obtained from DNA of cells, tissue or other test samples by standard methods. This standard methodology is found in references such as Sambrook, Fritsch and Maniatis, Molecular Cloning: A Laboratory Manual, 1989, p 9.16 - 9.19.

The US 6 265 171 (Herman et al.) describes a method for detecting a methylated CpG-containing nucleic acid present in a specimen by contacting the specimen with an agent that modifies unmethylated cytosine and amplifying the CpG-containing nucleic acid using CpG-specific oligonucleotide primers.

The WO 01/44504 concerns a method for detecting methylated CpG-containing nucleic acid by, contacting a nucleic acid containing specimen with an agent that modifies unmethylated cytosine. The method is particularly useful for prognosing, diagnosing or monitoring diseases associated with the methylation of nucleic acid, for example, tumours and certain developmental disorders.

The WO 00/70090 discloses an improved high-throughput and quantitative process for determining methylation patterns in genomic DNA samples based on amplifying modified nucleic acid, and detecting methylated nucleic acid based on amplification-dependent displacement of specifically annealed hybridization probes.

An in-tube methylation assay is described by Worm, Jesper et al., "In-tube DNA methylation profiling by flourescence melting curve analysis", Clinical Chemistry, vol. 47, no. 7, July 2001, pages 1183-1189 that intergrates amplification of bisulfite-treated DNA and melting analysis by using a thermal cycler coupled to a flourometer (LightCycler).

A method of making a set of labelled compounds by the use of a preferably particulate support is described in the WO 99/60007.

The WO 97/37041 describes a method to sequence DNA. The method utilizes the Sanger sequencing strategy and assembles the sequence information by analysis of the nested fragments obtained by base-specific chain termination via their different molecular masses using mass spectrometry, as for example, MALDI or ES mass spectrometry.

The synthesis and characterization of photocleavable peptide-DNA conjugates is described by Olejnik, J. et al.; "Photocleavable peptide-DNA conjugates: synthesis and applications to DNA analysis using MALDI-MS", Nucleic Acids Research, Oxford University Press, Surrey, GB, vol. 27, no. 23, 1 december 1999, pages 4626-4631, along with their use as photocleavable mass marker (PCMM) hybridisation probes for the detection of target DNA sequences by matrix-assisted laser desorption/ionisation (MALDI) mass spectrometry.

Accordingly, a great number of methods for methylation analysis are prior art. The present invention, however, shall solve the problem that the current methods cannot, that is, to amplify the DNA of interest or the DNA to be investigated, which in the following will be called the "target DNA" which is found in body fluids or serum, in a targeted manner, when other DNA segments of homologous sequence from another origin are present at the same time.

Generally, the target DNA as well as the otherwise present nucleic acids, which in the following are named background DNA, are amplified to the same extent, since the primers utilised cannot distinguish between target DNA and background DNA. However, a possibility to differentiate these DNAs results from their different methylation patterns. A method commonly used for this purpose is the methylation-sensitive PCR, abbreviated MSP (Herman JG, Graff JR, Myohanen S, Nelkin BD, Baylin SB. (1996), Methylation-specific PCR: a novel PCR assay for methylation status of CpG islands. Proc Natl Acad Sci U S A. Sep 3;93(18):9821-6). This method consists of several sub-steps. First, a bisulfite treatment corresponding to the prior art is carried out, which in turn provides that all cytosine bases are converted to uracil, while the methylated cytosine bases (5-methylcytosine) remain unchanged. In the next step, primers are used, which are completely complementary to a methylated DNA converted with bisulfite, but not to a corresponding DNA which was originally present unmethylated. When a PCR is conducted with such a primer, this leads to the circumstance that only the originally methylated DNA is amplified. Accordingly it is possible to use a primer, which in contrast only amplifies the unmethylated DNA. In this manner exclusively the DNA fragments to be investigated will be selectively produced, while the DNA to be analyzed as well as the background DNA are present, given that they are distinguishable from the background DNA with respect to their methylation state in a CpG position. The prior art is to infer the methylation state or the presence of a target DNA from the detection of such a DNA molecule to be investigated,
which in turn essentially permits a diagnosis, for example, of a tumor disorder in patients, since it is known that, for example, the serum DNA concentration is increased, in part drastically, in tumor patients. Only the DNA originating from the tumors shall then be detected, aside from the background DNA. In principle the DNA analysis in other body fluids is comparable.

The method described here, which is considered to be the closest prior art, however, has several disadvantages. For example, it is not possible to infer the quantity of the target DNA that is present in serum from the fact that an amplified fragment of this DNA can be detected. Even minimal quantities of such DNA are sufficient in order to obtain a positive result, which is an advantage, on the one hand, but can also be a great disadvantage, if one wants to evaluate, for example, the effect of a tumor resection on the serum DNA. The greatest difficulty, however, is that there are many CpG positions, in which the target DNA and the background DNA are only slightly distinguished with respect to their methylation state. It is obvious that the existing MSP method can only be conducted when one knows that the background DNA is definitively distinguished and is up to 100% different in the respective CpG position from the target DNA, if one does not want to risk false positive results. In contrast, if it is typical in a tumor tissue that e.g., in 95% of the tumor cells a specific position is present methylated, while in the otherwise present background DNA only a maximum of 5% is present methylated, then with the MSP method it is not possible to produce informative results, since a quantification of the template DNA by means of PCR is essentially not possible or is possible only with increased expenditure. This invention is also based on the knowledge that there are often patterns of methylation states in a DNA fragment, which are typical for a specific type of cells, for example, a tumor cell.

The prior art is also a method developed by Epigenomics, which amplifies target DNA and background DNA to the same extent after bisulfite treatment and then investigates the former CpG positions contained in the fragment by hybridization techniques, [or] alternatively by means of mini-sequencing or other current methods. This has the advantage that one obtains a quantitative pattern with respect to the investigated methylation positions, i.e., it produces a determination of the degree of methylation for a plurality of positions, which enables a very precise classification, e.g., in the case of solid tumors. The disadvantage of this method, however, is that it cannot supply accurate information in cases of excessive background DNA, since exactly this DNA is amplified along with the target DNA and both are analyzed in the mixture. This problem does not exist in the analysis of solid tumors, where one can select the material to be investigated in a targeted manner, but it can complicate the analysis of, for example, serum DNA.

The object of the present invention is now to overcome the disadvantages of the prior art and to combine the advantages of both methods [described above] for the detection in body fluids and serum. Thus, there is a particular emphasis on the detection of small quantities of unmethylated DNA in the presence of a comparatively large quantity of background DNA, a problem that has not yet been solved.

This task is solved by creating a method for detection of cytosine methylation in DNA samples, in which the following steps are conducted:
- a genomic DNA sample, which comprises DNA to be investigated (target DNA) and background DNA, is chemically treated such that all unmethylated cytosine bases are converted to uracil, while the 5-methylcytosine bases remain unchanged;
- the chemically treated DNA sample is amplified with the use of
   - at least two primer oligonucleotides, as well as
   - a polymerase, and
   - a nucleotide mixture containing
      either
      - 2'-deoxyguanosine triphosphate (dGTP), and
      - 2'-deoxyadenosine triphosphate (dATP), and
      - 2'-deoxythymidine triphosphate (dTTP) or 2'-deoxyuridine triphosphate (dUTP), .
      - and 2'-deoxycytidine triphosphate (dCTP), at art initial concentration which is at most half as much as the average initial concentration of the other three nucleotides for the amplification;
      or
      - 2'-decxycytidine triphosphate (dCTP), and
      - 2'-deoxyadenosine triphosphate (dATP), and
      - 2'-deoxythymidine triphosphate (dTTP) or 2'-deoxyuridine triphosphate (dUTP)
      - and 2'-deoxyguanosine triphosphate (dGTP), at an initial concentration which is at most half as much as the average initial concentration of the other three nucleotides for the amplification; and
- the methylation state in the target DNA is concluded from the presence or quantity of an amplificate.

A further method for the detection of cytosine methylation in DNA samples comprises the following steps. a genomic DNA sample, which comprises target DNA and background DNA, is chemically treated such that all unmethylated cytosine bases are converted to uracil, while the 5-methylcytosine bases remain unchanged;
the chemically treated DNA sample is amplified with the use of at least 2 primer oligonucleotides as well as a polymerase and a nucleotide mixture, the composition of which leads to a preference for the target DNA over the background DNA as the template;
and the amplificates are analyzed and the methylation state in the target DNA is concluded from the presence of an amplificate or its quantity.

In a variant of the method according to the invention, the nucleotide mixture only contains 2'-deoxyguanosine triphosphate (dGTP), 2'-deoxyadenosine triphosphate (dATP) and 2'-deoxythymidine triphosphate (dTTP). However, it is also preferred that the nucleotide mixture additionally contains a comparatively small concentration of 2'-deoxycytidine triphosphate (dCTP). The initial concentration of the dCTP is particularly preferred to be at most half as much as the average initial concentration of the other three nucleotides for the amplification.

In a further variant of the method, the nucleotide mixture contains only 2'-deoxycytidine triphosphate (dCTP), 2'-deoxyadenosine triphosphate (dATP) and 2'-deoxythymidine triphosphate (dTTP). However, it is also preferred that the nucleotide mixture additionally contains a comparatively small concentration of 2'-deoxyguanosine triphosphate (dGTP). The initial concentration of the dGTP is particularly preferred to be at most half as much as the average initial concentration of the other three nucleotides for the amplification.

In a variant of the method, 2'-deoxyuridine triphosphate is used instead of 2'-deoxythymidine triphosphate.

In another particularly preferred variant of the method, terminating dideoxynucleotides are additionally used.

It is also particularly preferred that the denaturing temperature lies below 90°C in the PCR amplification.

It is preferred according to the invention that the DNA samples are obtained from serum, plasma, urine, sputum or other body fluids of an individual.

It is additionally preferred according to the invention, that the DNA samples are obtained from cell lines, blood, sputum, stool, urine, serum, plasma, cerebrospinal fluid, tissue embedded in paraffin, for example, tissue from eyes, intestine, kidney, brain, heart, prostate, lung, breast or liver, histological slides and all possible combinations thereof.

It is most particularly preferred according to the invention that the chemical treatment is conducted with a bisulfite (= disulfite, hydrogen sulfite). It is also preferred that the chemical treatment is conducted after embedding the DNA in agarose. It is also and additionally preferred that in the chemical treatment, a reagent that denatures the DNA duplex and/or a radical scavenger are present.

It is preferred that the amplification in the second step is conducted in the presence of at least one other oligonucleotide or a PNA oligomer, which binds to a 5'-CG-3' dinucleotide or a 5'-tG-3'-dinucleotide or a 5'-Ca-3' dinucleotide, whereby this other oligonucleotide or PNA oligomer preferably binds to the background DNA and adversely affects its amplification and wherein "t" represents a thymine at a position which correlates with an unmethylated cytosine prior to bisulfite treatment and "a" correlates with such a thymine position.

It is particularly preferred that this binding site of the other oligonucleotide or PNA oligomer overlaps with the binding sites of the primers on the background DNA and this other oligonucleotide impedes the binding of at least one primer oligonucleotide to the background DNA.

It is again particularly preferred that at least two other oligonucleotides or PNA oligomers are used, whereby their binding sites again each overlap with the binding site of a primer to the background DNA and these other oligonucleotides and/or PNA oligomers impede the binding of both primer oligonucleotides to the background DNA

In addition, it is particularly preferred that one of these other oligonucleotides and/or PNA oligomers impedes the binding of the forward primer, while the other one impedes the binding of the reverse primer.

It is particularly preferred that these other oligonucleotides and/or PNA oligomers are present in at least five times the concentration of the primer oligonuleotides.

In addition, it is preferred according to the invention that the chemically treated DNA sample is amplified in the second step with the use of at least 2 primer oligonucleotides and another oligonucleotide, which hybridizes to a 5'-CG-3' dinucleotide or a 5'-tG-3' dinucleotide or a 5'-Ca-3' dinucleotide, and at least one reporter oligonucleotide, which hybridizes to a 5'-CG-3' dinucleotide or a 5'-tG-3' dinucleotide or a 5'-Ca-3' dinucleotide, as well as a polymerase; wherein the additional oligonucleotide preferably binds to the background DNA and adversely affects its amplification, and wherein the reporter oligonucleotide preferably binds to the target DNA and indicates its amplification and wherein "t" represents a thymine at a position which correlates with an unmethylated cytosine prior to bisulfite treatment and "a" correlates with such a thymine position. It is therefore advantageous that in addition to the reporter oligonucleotide, another oligomer which is labeled with a fluorescent dye is used, which hybridizes right next to the reporter oligonucleotide and this hybridization can be detected by means of fluorescence resonance energy transfer. In addition, it is advantageous that a Taqman assay is conducted. It is also preferred that a LightCycler assay is conducted. It is also preferred that an assay is conducted with the use of Molecular Beacons.

In another particularly preferred variant of the method, the other oligonucleotides and/or PNA oligomers bind to the background DNA and thus impede the complete primer oligonucleotide extension in the polymerase reaction. It is again particularly preferred that the polymerase used does not have 5'-3' exonuclease activity. Another preferred variant is that the other oligonucleotides are present in modified state at the 5'-end and thus cannot be significantly decomposed (degraded) by a polymerase with 5'-3' exonuclease activity.

In a particularly preferred variant, the primers themselves differentiate the target DNA from the background DNA. In an again particularly preferred variant of the method, the background DNA is methylated, while the target DNA is unmethylated, each at positions at which at least one amplification primer binds , whereby the one or more primers preferably bind to the target DNA. If the primers are to amplify preferably the target DNA, then these preferably contain TG or CA sequences at positions which corresponded to CG sequences prior to the bisulfite treatment. In this way, the primers do not amplify the previously methylated sequences that still contain CG and thus under these conditions ideally do not amplify background DNA. This strengthens the effect brought about by the nucleotide mixture of the preference for the unmethylated target DNA in the amplification.

It is particularly preferred according to the invention that additionally at least one reporter oligonucleotide, whose fluorescent properties change as a consequence of the amplification, is used in the amplification. In an again particularly preferred variant of the method, a Taqman assay or a LightCycler assay or an assay with the use of Molecular Beacons is conducted.

It is further preferred according to the invention that the oligonucleotides used in addition to the primers do not have a 3'-OH group. In addition, it is preferred that the reporter oligonucleotides bear at least one fluorescent label. It is also preferred that the reporter molecules indicate the amplification either by an increase or a decrease in the fluorescence. It is particularly advantageous that the increase or the decrease in the fluorescence is also used directly for the analysis and a conclusion of the methylation state of the DNA to be analyzed is made from the fluorescent signal.

It is further preferred according to the invention that the background DNA is present in 100X the concentration in comparison to the target DNA. It is further preferred that the background DNA is present in 1000X the concentration in comparison to the target DNA.

It is again preferred that a conclusion is made on the presence of a disease or another medical condition of the patient from the methylation degree of the different CpG positions investigated.

The term medical condition is meant to describe all those conditions of human nature that differ from a condition which the plurality of people, with a medical background, such as medicinal doctors, dentists, examiners, nurses etc. would call healthy. A medical condition would encompass all diseases, inherited physical defects of the human body and behavioural disorders, but also cancer diseases; CNS malfunctions, damage or disease; symptoms of aggression or behavioral disturbances; clinical, psychological and social consequences of brain damage; psychotic disturbances and personality disorders; dementia and/or associated syndromes; cardiovascular disease, malfunction and damage; malfunction, damage or disease of the gastrointestinal tract; malfunction, damage or disease of the respiratory system; lesion, inflammation, infection, immunity and/or convalescence; malfunction, damage or disease of the body as a consequence of an abnormality in the development process; malfunction, damage or disorder of the skin, the muscles, the connective tissue or the bones; endocrine and metabolic malfunction, damage or disease; headaches or sexual malfunction.

It is advantageous that the amplificates themselves are provided with a detectable label for the detection. It is again advantageous that the labels are fluorescent labels or/and that the labels are radionuclides or/and that the labels are removable mass labels, which are detected in a mass spectrometer.

It is further preferred that in the amplification, one of the primers is bound to a solid phase.

It is also [preferred] according to the invention that all of the amplificates are detected in the mass spectrometer and are thus clearly characterized by their mass.

The method of the present invention may be used for the diagnosis and/or prognosis of adverse events for patients or individuals, whereby these adverse events belong to at least one of the following categories: undesired drug interactions; cancer diseases; CNS malfunctions, damage or disease; symptoms of aggression or behavioral disturbances; clinical, psychological and social consequences of brain damage; psychotic disturbances and personality disorders; dementia and/or associated syndromes;
cardiovascular disease, malfunction and damage; malfunction, damage or disease of the gastrointestinal tract; malfunction, damage or disease of the respiratory system; lesion, inflammation, infection, immunity and/or convalescence; malfunction, damage or disease of the body as [a consequence of] an abnormality in the development process; malfunction, damage or disorder of the skin, the muscles, the connective tissue or the bones; endocrine and metabolic malfunction, damage or disease; headaches or sexual malfunction.

The use of a method according to the invention is also preferred for distinguishing cell types or tissues or for investigating cell differentiation.

The use of a method according to the invention is also preferred for the classification of patients into subgroups.

The subject of the present invention is also a kit consisting of a reagent containing:
- a bisulfit,
- primers for the amplification, and
- a nucleotide mixture, containing
   either
   - 2'-deoxyguanosine triphosphate (dGTP), and
   - 2'-deoxyadenosine triphosphate (dCTP), and
   - 2'-deoxythymindine triphosphate (dTTP) or 2'-deoxyuridine triphosphate (dCTP),
   - and 2'-deoxycytidine triphosphate (dCTP), at an initial concentration which is at most half as much as the average initial concentration of the other three nucleotides for the amplification;
   or
   - 2'-deoxycytidine triphosphate (dCTP), and
   - 2'-deoxyadenosine tripohsphate (dATP), and
   - 2'-deoxythymidine triphosphate (dTTP) or 2'-deoxyuridine triphosphate (dUTP),
   - and 2'-deoxyguancsine triphosphate (dGTP), at an initial concentration which is at most half as much as the average initial concentration of the other three nucleotide for the amplification.

A kit according to the present invention may also consist of a reagent containing bisulfite, primers for the amplification and a nucleotide mixture, which contains either only 2'-deoxyguanosine triphosphate (dGTP), 2'-deoxyadenosine triphosphate (dATP) and 2'-deoxythymidine triphosphate (dTTP) and no dCTP, or only a relatively small concentration of 2'-deoxycytidine triphosphate (dCTP). It is particularly preferred that the concentration of dCTP is at most half as much as the average concentration of the other three nucleotides.

A further variant of the kit consists of a reagent containing bisulfite, primers for the amplification and a nucleotide mixture, which contains either only 2'-deoxycytidine triphosphate (dCTP), 2'-deoxyadenosine triphosphate (dATP) and 2'-deoxythymidine triphosphate (dTTP) and no dGTP, or only a relatively small concentration of 2'-deoxyguanosine triphosphate (dGTP). It is particularly preferred that the concentration of dGTP is at most half as much as the average concentration of the other three nucleotides.

The present invention thus describes a method for the detection of the methylation state of genomic DNA samples. In contrast to the methods which were known previously, the methylation- degree of a set of CpG positions is determined in a selected subgroup of DNA fragments, e.g., in serum, plasma, urine or sputum, so that an analysis is also possible in the presence of an excess of diagnostically irrelevant background DNA. With the method according to the invention the selective detection of unmethylated target DNA in the presence of an excess of diagnostically irrelevant, methylated background DNA is particularly successful. Up to now, this could not be shown with comparable methods.

In this way, the preferred method again consists of several steps, which can be summarized as follows:
First, serum and/or other body fluids are sampled from the patient and the DNA found therein is isolated insofar as necessary. Then in the second step, a chemical treatment, preferably with a bisulfite (= hydrogen sulfite, disulfite) is conducted, wherein, for example, all unmethylated cytosine bases are converted to uracil, but the methylated cytosine bases (5-methylcytosine) remain unchanged. In the third step of the method, an amplification is now conducted, in which, preferably the target DNA is amplified, but the background DNA is not, or is amplified only to a lesser extent. A particularly suitable nucleotide mixture, as described above, leads to the preference for the target DNA in the amplification, or at least contributes to this preference. The denaturing temperature is also particularly preferably adapted so that the originally unmethylated DNA is preferably melted, but the originally methylated DNA remains double-stranded. In the following, fourth step, the amplified fragments are now detected. It is also possible to analyze them in further detail based on their methylation signature and to determine the degree of methylation of several former CpG positions in the amplificates. In the fifth step of the method, the presence of a disease or another medical condition of the patient is concluded from the methylation degree of the different CpG positions investigated or from the presence of an amplificate or from the formed quantity of this amplificate alone.

The first step of the method, the obtaining of samples, is preferably conducted by sampling of body fluids, such as, e.g., sputum, urine, plasma or serum, but it is obvious that the method can be conducted with many different kinds of samples from different sources, which are listed here without any claim to completeness.

The genomic DNA used in the method is preferably obtained from a DNA sample, whereby sources for DNA include, e.g., cell lines, blood, sputum, stool, urine, serum, cerebrospinal fluid, tissue embedded in paraffin, for example tissue from eyes, intestine, kidney, brain, heart, prostate, lungs, breast or liver, histological slides and all possible combinations thereof.

A purification or concentration of the DNA is performed in several cases prior to the bisulfite treatment in order to avoid a disruption of the bisulfite reaction and/or the subsequent PCR by too high a content of impurities. It is known, however, that, for example, a PCR can be conducted from tissue, for example, after treatment with proteinase K without further purification, and this is true logically also for the bisulfite treatment and subsequent PCR.

The chemical treatment is preferably conducted by treatment with a bisulfite (= hydrogen sulfite, disulfite), more preferably sodium bisulfite (ammonium bisulfite is less suitable). The reaction is either conducted according to a published variant, preferably the DNA is embedded in agarose in order to keep the DNA in the single-stranded state during treatment, or, according to a new variant, by treatment in the presence of a radical scavenger and a denaturing reagent, preferably an oligoethylene glycol dialkyl ether or, for example, dioxane. Prior to the PCR reaction, the reagents are removed either by washing in the case of the agarose method or by a DNA purification method (prior art, precipitation or binding to a solid phase, membrane) or, the reagents are brought to a concentration range which no longer significantly influences the PCR, simply by dilution.

In the third step of the method, an amplification of the bisulfite-treated DNA is conducted. The amplification method herein prefers the target DNA over the background DNA. Such a preference is achieved by variation of the nucleotide concentration in the PCR. Even with equal concentration of all 4 nucleotides in the PCR an unequal amplification of the previously methylated and unmethylated DNA can occur. Even when this preference is only slight in one amplification cycle in a PCR reaction, a considerable bias can result in amplification efficiency after the usual 30-40 cycles. The basic idea of the present invention is now to make use of this generally undesired effect by sharpening the preference for the target DNA over the background DNA by adaptation of the PCR conditions. The method according to the invention is thus particularly suitable for the preferred amplification of unmethylated DNA in the presence of methylated background DNA. The relative concentration of the nucleotides relative to one another will be changed here particularly in order to achieve such a bias in favor of the originally unmethylated DNA. If unmethylated DNA was treated with bisulfite, then after the amplification a fragment is produced, which can contain far fewer CG base pairs than a fragment that was generated from a corresponding methylated DNA. A preference for the unmethylated DNA is thus achieved in the present invention by the fact that essentially less dCTP and/or dGTP than dATP and dTTP is added to the PCR reaction. Due to the fact that the dCTP and dGTP nucleotides are thus limiting in the PCR, the target DNA, which contains less C and G, is preferred over the background DNA.

Analogously, a preference for the target DNA can also be achieved by modifying the nucleotide composition. Thus, e.g., terminating nucleotide triphosphates such as, for example, dideoxy derivates are utilized in small concentrations. These produce a reduced amplification efficiency in the polymerase chain reaction, due to the disruption of chain extension, but again the background DNA is more adversely affected in the amplification than the unmethylated target DNA, if, for example, ddCTP or ddGTP are selected as terminators.

Another adaptation of the PCR conditions can be produced by modifying the denaturing temperature. A lower denaturing temperature in turn promotes the originally unmethylated DNA, since this contains fewer C/G base pairs with otherwise the same sequence. The originally methylated DNA is thus melted only incompletely under these conditions and is not available as a template in the following amplification cycle.

The change in the denaturing temperature and the above-described changes in the nucleotide composition are particularly preferably utilized in combination, in order to finally suppress amplification of the background DNA practically completely over several cycles.

The method according to the invention preferably may also be used in combination with a suitable method (such as e.g., MSP) and methylation positions which are adapted for this and which also otherwise permit the selective amplification of the target DNA. The selection of the respective CpG positions herein is made according to the premise that they should distinguish as much as possible between the background DNA and the target DNA with respect to their methylation. For this purpose, preferably, first the methylation profiles are determined for those segments of a gene that are in question each time, both for tumors to be investigated as well as for background DNA of healthy individuals. Those positions, which have the greatest differences between tumor DNA and background DNA (for example, in serum), are selected as methylation positions, which will be distinguished by the named methods. Such positions are already known for a plurality of genes, for example, for GSTpi, for HIC-1 and MGMT (von Wronski MA, Harris LC, Tano K, Mitra S, Bigner DD, Brent TP. (1992) Cytosine methylation and suppression of O6-methylguanine-DNA methyltransferase expression in human rhabdomyosarcoma cell lines and xenografts. Oncol Res.;4(4-5):167-74; Esteller M, Toyota M, Sanchez-Cespedes M, Capella G, Peinado MA, Watkins DN, Issa JP, Sidransky D, Baylin SB, Herman JG. (2000), Inactivation of the DNA repair gene O6-methylguanine-DNA methyltransferase by promoter hypermethylation is associated with G to A mutations in K-ras in colorectal tumorigenesis. Cancer Res. May 1;60(9):2368-71).

After the selective amplification of the target DNA, the methylation state of several other CpG positions can now be determined preferably according to methods, which are known. If the amplification bias is taken into consideration, in this case, a confirmation of the result of the amplification can thus usually be obtained.

It is obvious, however, that even in this case, the formation of a PCR fragment itself can provide sufficient information in individual cases, given the situation -as it is also in MSP- that the position already investigated in the amplification is unmethylated practically up to 100%, for example, in the background DNA, but is methylated in the target DNA. In an individual case this may even be sufficient for a diagnosis.
It is preferred that in a PCR reaction, several fragments are generated simultaneously, i.e., that a multiplex PCR is conducted. Care must be taken in its design that not only the primers, but also possibly other oligonucleotides utilized should not be complementary to one another. However, in the case of bisulfite-treated DNA, one has the advantage that a forward primer can never function also as a reverse primer, due to the different G and C content of the two DNA strands, which facilitates multiplexing.

In the simplest case, the generated fragments are now detected. For this purpose, all possible known molecular biology methods are possible, such as gel electrophoresis, sequencing, liquid chromatography or hybridizations. These are also conceivable for the quality control of the preceding method steps. As indicated above, however, the subsequent analysis of the degree of methylation of other CpG positions is particularly preferred.

Detection techniques, which are particularly suitable for this, are hybridization to oligomer arrays and, for example, primer extension (minisequencing) reactions. Hybridization to oligomer arrays can be used without further change of protocols when compared with the closest prior art (Olek A, Olek S, Walter J; WO 99/28498). It is preferred, however, to hybridize the amplificates to an array of oligomers, which consists of pairs of oligonucleotides immobilized to a solid phase, one of which hybridizes most preferably to a DNA segment containing an originally unmethylated CpG and the other in turn hybridizes most preferably to the corresponding segment in which originally a methylated CpG was contained, each prior to the bisulfite treatment and amplification. In this case, the amplificate or the amplificates are particularly preferably labeled fluorescently or radioactively or with removable mass tags, so that after the hybridization, the fragments bound to both oligonucleotides of a pair can be detected and quantified on the basis of this label. An intensity ratio is obtained, from which the degree of methylation at the respective CpG position can be determined, for example, after calibration of the experiment with completely methylated and completely unmethylated DNA, whereby here again the bias in the amplification has to be considered. A plurality of fragments can be detected simultaneously on such an oligomer array. It makes sense and it is preferred that the array also contains oligomers for the experimental controls, because that way , for example, the ratio of the target DNA, entering the analysis, to the background DNA, can be determined.

Primer extension reactions can also be conducted on oligonucleotides immobilized on a solid phase. Although not absolutely necessary, the immobilizing of these primers is preferred, since usually a plurality of CpG positions from several amplificates will be investigated and this can be conducted on a solid phase, thus on an oligomer array, significantly more easily and in one experiment. It is particularly preferred that the primers are found directly next to a CpG position to be investigated and that the extension occurs only by one nucleotide. It is particularly preferred that only dideoxythymidine and dideoxycytidine are added as nucleotides and that these are each labeled with a different fluorescent dye, whereby, of course, other distinguishable labels such as mass tags are also conceivable and preferred. After a bisulfite treatment and amplification, previously methylated CGs are present as CGs and unmethylated CGs are now present as TGs. The primer extension reaction thus leads to the incorporation of either a dideoxycytidine or a dideoxythymidine. The methylation of the respective position can be concluded from the ratio of the fluorescent labels detected each time for these two terminators. It is also possible and preferred in this case, to conduct the primer extension with deoxycytidine and deoxythymidine, if one does not add any guanine derivative, and consequently for a TG or CG sequence the primer extension terminates already after one base anyway. In addition, it is also preferred to conduct the analysis analogously on the corresponding strand by distinguishing CA and CG, then accordingly proceeding with dideoxy-ATP und dideoxy-GTP or their derivatives. The selection of the positions and the selection of terminators and nucleotides in the primer extension reaction must be adapted to the selection of nucleotides and reaction conditions in the amplification according to the invention. Meaningful and meaningless combinations will be obvious to the person skilled in the art.

A particularly preferred variant of the method is the analysis of CpG positions by use of Taqman or LightCycler technology variants directly during the amplification. In this way, additional fluorescently labeled oligonucleotides are added to the oligonucleotides which are provided for the amplification of the target DNA, and the change in fluorescence during the PCR reaction is measured. Since the target DNA is amplified predominently, information on the methylation state of different CpG positions is obtained for the most part directly from this change in fluorescence. Since different oligonucleotides are preferably each provided with a different fluorescent dye, a differentiation of the change in fluorescence during the PCR is also possible separately for the different positions and fragments.

This change in fluorescence that is dependent on the methylation state can be obtained by numerous methods, two of which will be introduced here by way of example.

First of all, oligonucleotide probes can be used preferably, which bind specifically either to a sequence which is produced by chemical treatment of an unmethylated DNA at the corresponding position, or to a sequence which is produced by chemical treatment of a methylated DNA at the corresponding position. These probes are particularly preferably provided with two fluorescent dyes, a quencher dye and a fluorescent dye serving as a marker. Both are coupled to these oligonucleotide probes. Now if a PCR reaction occurs with the target DNA as the template, then the PCR reaction is blocked this time by the fluorescently-labeled oligomer probes. However, since this is not resistant to the nuclease activity of the polymerase, a decomposition of the probe bound to the template DNA occurs during the PCR reaction, which correlates with the binding efficiency of the probe to the template, since the unbound probe is not decomposed by the polymerase. The decomposition of the probe is now directly visible by an increase of the fluorescence of the marker dye, because the quencher dye and the fluorescent dye serving as the marker are separated from one another. In principle, this involves a variant of the so-called Taqman assay.

Accordingly, what is measured is the formation of a PCR product from the target DNA, but only if the investigated position is also present in the methylation state, that the probe can detect by hybridization to the chemically treated DNA. A cross-check with a probe that would bind correspondingly to the respective CpG position in the other methylation state is thus appropriate and preferred.

Different fluorescent dyes with different emission wavelengths for several probes are preferably utilized together with the quencher, in order to be able to distinguish among the probes and thus to achieve a multiplexing.

In such an assay, oligonucleotides binding to the respective CpG position are utilized, which prevent a significant amplification of the background DNA. The amplification of the target DNA can also be analyzed in such a way that one investigates the same position also with a probe such as described above and detects the amplification accordingly by a probe binding to the respective CpG position. In this case, it is particularly preferred that the oligonucleotide that cannot be decomposed binds selectively to the background DNA, while the fluorescently labeled probe binds to the target DNA. In a particularly preferred variant of the method, the probe and the oligonucleotide that cannot be decomposed have the same sequence, except for one, but no more than two nucleobases.

It is also preferred that several positions can be simultaneously investigated with one probe for their degree of methylation.

If a more precise quantification of the degree of methylation of a position is desired, then two probes competing with one another and having different dyes can also be preferably utilized, whereby one of these again preferably binds in the case of an unmethylated position in the target DNA, while the other preferably binds in the case of a methylated position. The methylation state of the investigated position as well as the successful conducting of the amplification specific to the target DNA can then again be concluded from the ratio of the increases in fluorescence for the two dyes.

A basically different method, in which, however, there is also a change in fluorescence during the PCR, is known presently as LightCyder^{™} technology. The fact is utilized that a fluorescence resonance energy transfer (FRET) can only occur between two dyes, if these are spaced in the immediate vicinity to one another, i.e., within 1-5 nucleotides. Only then can the second dye be excited by the emission of the first dye, and then in its turn, emit light of another wavelength, which is then detected.

In the present case of methylation analysis, a hybridization of a fluorescently labeled probe to the respective chemically treated DNA occurs at a CpG position, and the binding of this probe depends in turn on whether the target DNA was methylated or unmethylated at this position prior to the bisulfite treatment. Another probe with another fluorescent dye binds directly adjacent to this probe. This binding preferably occurs in turn as a function of methylation, if another methylatable position is present in the respective sequence segment. During the amplification, the DNA is now amplified, for which reason continuously more fluorescently labeled probes bind adjacent to the position in question, insofar as these have the methylation state necessary for this, and thus an increasing FRET is measured.

A multiplexing with several different fluorescently labeled probes is also produced preferably by this method.

In summary, a method is particularly preferred for the detection of cytosine methylation in DNA samples, in which the following steps are conducted: First, a genomic DNA sample, which comprises the target DNA and background DNA, is chemically treated such that all unmethylated cytosine bases are converted to uracil, while the 5-methylcytosine bases remain unchanged; then the chemically treated DNA sample is amplified with the use of at least 2 primer oligonucleotides as well as a polymerase, whereby the target DNA is preferred over the background DNA as a template; and in the next step, the amplificates will be analyzed and the methylation state in the target DNA will be concluded from the presence of an amplificate and/or from the analysis of additional positions.

In a particularly preferred variant of the [method] according to the invention, the nucleotide mixture only contains 2'-deoxyguanosine triphosphate (dGTP), 2'-deoxyadenosine triphosphate (dATP) and 2'-deoxythymidine triphosphate (dTTP). However, it is also preferred that the nucleotide mixture additionally contains a comparatively small concentration of 2'-deoxycytidine triphosphate (dCTP). The initial concentration of the dCTP is then particularly preferred to be at most half as much as the average initial concentration of the other three nucleotides for the amplification.

In an also particularly preferred variant of the method, the nucleotide mixture contains only 2'-deoxycytidine triphosphate (dCTP), 2'-deoxyadenosine triphosphate (dATP) and 2'-deoxythymidine triphosphate (dTTP). However, it is also preferred that the nucleotide mixture additionally contains a comparatively small concentration of 2'-deoxyguanosine triphosphate (dGTP). The initial concentration of the dGTP is then particularly preferred to be at most half as much as the average initial concentration of the other three nucleotides for the amplification.

In another, particularly preferred variant of the method, the denaturing temperature in the PCR is changed so that the target DNA is melted, but not the background DNA.

In a particularly preferred variant of the method, the analysis or the further analysis is conducted by means of hybridization on oligomer arrays, wherein oligomers can be nucleic acids or molecules such as PNAs that are similar in their hybridization properties. The oligomers preferably hybridize to the DNA to be analyzed over a 12-22 base long segment and comprise a CG, TG or CA dinucleotide. Preferably, the methylation states of more than 20 methylation positions of the target DNA are detected in one experiment with this method, and it is particularly preferred that more than 60 methylation positions are detected.

A method is also particularly preferred, in which the further analysis is conducted by measuring the length of the amplified target DNA, whereby methods for length measurement comprise gel electrophoresis, capillary gel electrophoresis, chromatography (e.g. HPLC), mass spectrometry and other suitable methods.

A method is also particularly preferred, in which the further analysis is conducted by sequencing, whereby methods for sequencing comprise the Sanger method, the Maxam-Gilbert method, and other methods such as sequencing by hybridization (SBH). Again, a method is preferred, whereby the sequencing (according to Sanger) is carried out for each CpG position or a small group of CpG positions, each with a separate primer oligonucleotide and the extension of the primer constitutes only one or just a few bases, and the methylation states of the respective positions in the target DNA are concluded from the type of primer extension.

In a particularly preferred variant of the method, a conclusion is made on the presence of a disease or another medical condition of the patient from the methylation degree of the different CpG positions investigated

it is particularly preferred that the amplificates themselves are also provided with a detectable label for the detection. The labels preferably involve fluorescent labels, radionuclides, or removable mass labels, which are detected in a mass spectrometer.

In addition, a method is preferred in which one of the primers is bound to a solid phase in the amplification.

A variant of the method is also preferred, wherein all the amplificates are detected in the mass spectrometer and are thus clearly characterized by their mass.

The method of the present invention may be used for the diagnosis and/or prognosis of adverse events for patients or individuals, whereby these adverse events belong to at least one of the following categories: undesired drug interactions; cancer diseases; CNS malfunctions, damage or disease; symptoms of aggression or behavioral disturbances; clinical, psychological and social consequences of brain damage; psychotic disturbances and personality disorders; dementia and/or associated syndromes;
cardiovascular disease, malfunction and damage; malfunction, damage or disease of the gastrointestinal tract; malfunction, damage or disease of the respiratory system; lesion, inflammation, infection, immunity and/or convalescence; malfunction, damage or disease of the body as [a consequence of] an abnormality in the development process; malfunction, damage or disorder of the skin, the muscles, the connective tissue or the bones; endocrine and metabolic malfunction, damage or disease; headaches or sexual malfunction.

The use of one of the described methods is also preferred for distinguishing cell types or tissues or for investigating cell differentiation.

A kit may also consist of a reagent containing bisulfite, primers and a nucleotide mixture for producing the amplificates, as well as, optionally, instructions for conducting at least one of the described method variants.

The kit consists of at least three reagents, the bisulfite reagent, the nucleotide mixture as well as the primers that can be included individually or also as a mixture.

The nucleotide mixture preferably comprises only 2'-deoxyguanosine triphosphate (dGTP), 2'-deoxyadenosine triphosphate (dATP) and 2'-deoxythymidine triphosphate (dTTP). However, it is also preferred that the nucleotide mixture additionally contains a comparatively small concentration of 2'-deoxycytidine triphosphate (dCTP). It is particularly preferred that the concentration of dCTP is at most half as much as the average concentration of the other three nucleotides.

The nucleotide mixture also preferably comprises only 2'-deoxycytidine triphosphate (dCTP), 2'-deoxyadenosine triphosphate (dATP) and 2'-deoxythymidine triphosphate (dTTP). However, it is also preferred that the nucleotide mixture additionally contains a comparatively small concentration of 2'-deoxyguanosine triphosphate (dGTP). It is particularly preferred that the concentration of dGTP is at most half as much as the average concentration of the other three nucleotides.

The nucleotide mixture can also contain dideoxynucleotides. The kit may also contain buffer components for the amplification and/or the bisulfite reaction.

The following examples explain the invention:

### Example 1: Production of the template DNA and establishing a methylation-sensitive PCR for GSTp1

Human DNA from peripheral blood (Promega, Madison USA), which is untreated and methylated in vitro enzymatically, and which has then been subjected to a bisulfite treatment, as well as corresponding unmethylated DNA, which has also been subjected to a bisulfite treatment, serve as the template DNAs. For the methylation of all CG dinucleotides, 6 µg of DNA in a reaction volume of 150 µl were reacted with Sssl (New England Biolabs, Frankfurt/Main) according to the manufacturer's instructions. The bisulfite treatment was conducted according to a published method (Olek A, Oswald J, Walter J. A modified and improved method for bisulphate based cytosine methylation analysis Nucleic Acids Res. 1996 DEC 15;24(24):5064-6).

A 153-bp GSTp1 fragment (positions 1242-1393 in Sequence Acc. No. M24485.1) was amplified with the bisulfite-DNA specific primers 2cf GTTTT(CT)GTTATTAGTGAGT and 2cr TCCTAAATCCCCTAAACC in a reaction volume of 25 µl (1x reaction buffer, Qiagen; 1 U HotstarTaq, Qiagen; 200 µM of each dNTP, except for 75 µM dCTP, 500 nM of each primer, 0.05-10 ng of bisulfite-treated template DNA) under the following PCR conditions (95°C - 15 min; 46 cycles: 88.5°C - 0:45 min, 52°C - 0:45 min, 72°C - 0:20 min; 72°C - 10 min). By sequencing the GSTp1 fragments, it could be shown that human DNA from peripheral blood does not have methylated CG dinucleotides in this fragment, while, on the other hand, all CG dinucleotides are present in the methylated form in the Sssl-treated DNA. It can also be shown that the PCR proceeds clearly more efficiently for the unmethylated DNA with the same initial concentration than for the methylated DNA, for which practically no detectable product was obtained under the selected conditions.

### Example 2: GSTP1 PCR with different dNTP mixes on bisulfite treated methylated and unmethylated DNA templates.

Genomic DNA was isolated from the samples and treated with a solution of bisulfite as it is described in Olek et al. Nucleic Acids Res. 1996 Dec 15;24(24):5064-6. As a result of this treatment cytosine bases that were unmethylated were converted to thymine and are in the following indicated as such by the use of small t instead of capital T which respectively stands for a thymine base, that was a thymine base prior to treatment with bisulfite. It can be calculated that the nucleotide composition of the GSTP1 (Exon1) PCR amplificate (nt 1183 to nt 1309 in Genbank Accession M24485.1) generated on bisulfite treated methylated and bisulfite treated unmethylated template DNA is significantly different. The GSTp1 fragment amplified from bisulfite treated methylated DNA bears a G+C content of 46% and the fragment amplified from bisulfite treated unmethylated DNA bears a G+C content of 35%. Therefore, the GSTp1 PCR performance on a bisulfite treated mixture or combination of methylated and unmethylated DNA is dependent on the concentration of the different dNTPs (dATP, dTTP, dGTP and dCTP) in the dNTP mix used in the PCR. This shall be demonstrated in the following:

The PCR of the GSTP1 (Exon 1) was performed in a total volume of 20 µl using a LightCycler device (Roche Diagnostics). The real time PCR reaction mix contained 10 µl of template DNA (for concentrations see below), 2 U of FastStart DNA polymerase (Roche Diagnostics, Penzberg), 1x reaction buffer (Roche Diagnostics, Penzberg), 0.25 mg/ml bovine serum albumin (Roche Diagnostics, Penzberg), 50-200 µM dNTP solution (Roche Diagnostics, Penzberg concentration of 200 µM dNTP (dATP, dTTP, dGTP and dCTP (50 µM each)), 1:80000 dilution of SybrGreen (MoBiTec, Göttingen), and 3mM MgCl₂. Thermocycling conditions began with a 95 degree C incubation for 10 minutes, then 55 cycles of the following steps: 95 degrees C for 10 seconds, 56 degrees C for 30 seconds, and 72 degrees C for 10 seconds. Fluorescence was detected after the annealing phase at 56 degrees C in each cycle.

As template DNA human DNA isolated from peripheral blood cells and commercially available enzymatically up-methylated human DNA (provided by Serologicals), which were both bisulfite treated, was used. The amount of DNA after bisulfite treatment was measured by UV absorption at 260nm. The performance of the assay on 100ng, 10ng, 1ng, 0.5ng, 0.25ng, 0.125ng and 0.0625ng bisulfite treated methylated template DNA was analyzed. Table 1 shows the mean of the cycle threshold values of 4 replicates as calculated by the LightCycler software. The data indicate, that the assays show a linearity of at least 4 orders of magnitude on bisulfite treated methylated template DNA. The absolute analytical sensitivity of the assay was found to be at least 25pg bisulfite treated methylated template DNA. There was no significant difference in PCR performance for the methylated compared with the unmethylated DNA, when all four nucleotides were used.

We expect, that the performance of the PCR on different methylated bisulfite treated template DNA is depending on the composition of the dNTP mix used in the PCR. Table 2 and 3 show the expected performance (lower threshold cycles indicate better performance) of GSTP1 PCR using 2 different dNTP mixes.

**Table 1 : Performance of GSTP1 (Exon 1) PCR with 200 µM dNTP (50 µM each nucleotide)**

| **template DNA (ng)** | **Mean of 4 threshold cycles obtained from 4 replicated experiments with methylated template DNA** | **Mean of 4 threshold cycles obtained from 4 replicated experiments with unmethylated template DNA** |
|---|---|---|
| 100 | 27.4 | 27.3 |
| 10 | 30.7 | 30.9 |
| 1 | 34.6 | 34.8 |
| 0.5 | 35.8 | 35.5 |
| 0.25 | 36.7 | 37.0 |
| 0.125 | 38.3 | 38.5 |
| 0.0625 | - | - |

**Table 2 : Performance of GSTP1 (Exon 1) PCR on bisulfit treated methylated DNA using different dNTP mixes**

| **methylated template DNA** | **Expected threshold cycles obtained from experiments with 60µM dGTP, 60µM dCTP, 40 µM dATP, 40µM TTP** | **Expected threshold cycles obtained from experiments with 40µM dGTP, 40µM dCTP, 60 µM dATP, 60µM TTP** |
|---|---|---|
| 100 | 27 | >>27 |
| 10 | 30 | >>30 |
| 1 | 33 | >>33 |
| 0.5 | 34 | >34 |
| 0.25 | 35 | >35 |
| 0.125 | 36 | >36 |

**Table 3 : Performance of GSTP1 (Exon 1) PCR on bisulfit treated unmethylated DNA using different dNTP mixes**

| **unmethylated template DNA** | **Expected threshold cycles obtained from experiments with 60µM dGTP, 60µM dCTP, 40 µM dATP, 40µM TTP** | **Expected threshold cycles obtained from experiments with 40µM dGTP, 40µM dCTP, 60 µM dATP, 60µM TTP** |
|---|---|---|
| 100 | >>27 | 27 |
| 10 | >>30 | 30 |
| 1 | >>3 | 33 |
| 0.5 | >34 | 34 |
| 0.25 | >35 | 35 |
| 0.125 | >36 | 36 |

## Claims

1. A method for the detection of cytosine methylation in DNA samples, **characterized in that** the following steps are conducted:
- a genomic DNA sample, which comprises DNA to be investigated (target DNA) and background DNA, is chemically treated such that all unmethylated cytosine bases are converted to uracil, while the 5-methylcytosine bases remain unchanged;
- the chemically treated DNA sample is amplified with the use of
- at least two primer oligonucleotides, as well as
- a polymerase, and
- a nucleotide mixture containing
either
- 2'-deoxyguanosine triphosphate (dGTP), and
- 2'-deoxyadenosine triphosphate (dATP), and
- 2'-deoxythymidine triphosphate (dTTP) or 2'-deoxyuridine triphosphate (dUTP),
- and 2'-deoxycytidine triphosphate (dCTP), at an initial concentration which is at most half as much as the average initial concentration of the other three nucleotides for the amplification;
or
- 2'-deoxycytidine triphosphate (dCTP), and
- 2'-deoxyadenosine triphosphate (dATP), and
- 2'-deoxythymidine triphosphate (dTTP) or 2'-deoxyuridine triphosphate (dUTP)
- and 2'-deoxyguanosine triphosphate (dGTP), at an initial concentration which is at most half as much as the average initial concentration of the other three nucleotides for the amplification; and
- the methylation state in the target DNA is concluded from the presence or quantity of an amplificate.

2. The method according to claim 1, further **characterized in that** terminating dideoxynucleotides are additionally used in the amplification.

3. The method according to one of the preceding claims, further **characterized in that** the denaturing temperature lies below 90°C in the PCR amplification.

4. The method according to one of the preceding claims, further **characterized in that** the sample DNA is obtained from serum, plasma, urine, sputum or other body fluids of an individual.

5. The method according to one of the preceding claims, further **characterized in that** the chemical treatment is conducted with a bisulfite, disulfite or hydrogen sulfite containing solution.

6. The method according to claim 5, further **characterized in that** the chemical treatment is conducted after embedding the DNA in agarose.

7. The method according to claim 5, further **characterized in that** in the chemical treatment, a reagent that denatures the DNA duplex and/or a radical scavenger is present.

8. The method according to one of the preceding claims, further **characterized in that** the amplification is conducted in the presence of at least one other oligonucleotide or a PNA oligomer, which binds to a 5'-CG-3' dinucleotide or a 5'-tG-3'-dinucleotide or a 5'-Ca-3' dinucleotide, whereby the other oligonucleotide or PNA oligomer preferably binds to the background DNA and adversely affects its amplification and wherein "t" represents a thymine at a position which correlates with an unmethylated cytosine prior to bisulfite treatment and "a" correlates with such a thymine position.

9. The method according to claim 8, further **characterized in that** this binding site of the other oligonucleotide or PNA oligomer overlaps with the binding sites of the primers on the background DNA, and said other oligonucleotide or PNA oligomer thus impedes the binding of at least one primer oligonucleotide to the background DNA.

10. The method according to one of claims 8 or 9, further **characterized in that** at least two other oligonucleotides or PNA oligomers are used, whereby their binding sites again each overlap with the binding site of a primer to the background DNA and said other oligonucleotides and/or PNA oligomers thus impede the binding of both primer oligonucleotides to the background DNA.

11. The method according to one of claims 8 to 10, further **characterized in that** these other oligonucleotides and/or PNA oligomers are present in at least five times the concentration of the primer oligonuleotides.

12. The method according to one of the preceding claims, further **characterized in that** the polymerase used has no 5'-3' exonuclease activity.

13. The method according to one of the preceding claims, further **characterized in that** the other oligonucleotides are modified at the 5'-end and thus cannot be significantly degraded by a polymerase with 5'-3' exonuclease activity.

14. The method according to one of the preceding claims, further **characterized in that** the primers in the amplification distinguish between target DNA and background DNA.

15. The method according to claim 14, further **characterized in that** the background DNA is methylated, while the target DNA is unmethylated, each at positions at which at least one primer for the amplification binds, whereby the one or more primers preferably bind to the target DNA after the chemical treatment.

16. The method according to one of the preceding claims, further **characterized in that** additionally at least one reporter oligonucleotide is used in the amplification whose fluorescence properties change as a consequence of the amplification.

17. The method according to claim 14, further **characterized in that** a Taqman assay or a LightCycler assay or an assay with the use of Molecular Beacons is conducted to conclude upon the methylation state at the last step of the method.

18. The method according to one of claims 16 or 17, further **characterized in that** the reporter oligonucleotide bears at least one fluorescent label.

19. The method according to one of claims 11 to 15, further **characterized in that** the reporter oligonucleotide or the reporter oligonucleotides indicates or indicate the amplification either by an increase or a decrease in fluorescence.

20. The method according to claim 19, further **characterized in that** the increase or decrease in fluorescence is used directly for the analysis and a conclusion on the methylation state of the DNA to be analyzed is made from the fluorescent signal.

21. The method according to one of the preceding claims, further **characterized in that** a conclusion is made on the presence of a disease or another medical condition of the patient from the methylation degree of the different CpG positions investigated.

22. The method according to one of the preceding claims, further **characterized in that** the amplificates themselves bear a detectable label for the detection.

23. The method according to claim 22, further **characterized in that** the labels are fluorescent labels.

24. The method according to claim 22, further **characterized in that** the labels are radionuclides.

25. The method according to claim 22, further **characterized in that** the labels are removable mass labels which are detected in a mass spectrometer.

26. The method according to one of the preceding claims, further **characterized in that** during amplification, one of the primers is bound to a solid phase.

27. The method according to one of claims 1 to 25, further **characterized in that** all the amplificates are detected in the mass spectrometer and are thus clearly **characterized by** their mass.

28. A kit consisting of a reagent containing:
- a bisulfit,
- primers for the amplification, and
- a nucleotide mixture, containing
either
- 2'-deoxyguanosine triphosphate (dGTP); and
- 2'-deoxyadenosine triphosphate (dATP), and
- 2'-deoxythymindine triphosphate (dTTP) or 2'-deoxyuridine triphosphate (dUTP),
- and 2'-deoxycytidine triphosphate (dCTP), at an initial concentration which is at most half as much as the average initial concentration of the other three nucleotides for the amplification;
or
- 2'-deoxycytidine triphosphate (dGTP), and
- 2'-deoxyadenosine tripohsphate (dATP), and
- 2'-deoxythymidine triphosphate (dTTP) or 2'-deoxyuridine triphosphate (dUTP),
- and 2'-deoxyguanosine triphosphate (dGTP), at an initial concentration which is at most half as much as the average initial concentration of the other three nucleotides for the amplification.

## Patentansprüche

1. Verfahren zum Nachweis von Cytosinmethylierung in DNA-Proben, **dadurch gekennzeichnet, dass** die folgenden Schritte ausgeführt werden:
- eine genomische DNA-Probe, welche zu untersuchende DNA (Target-DNA) und Hintergrund-DNA umfasst, wird chemisch derart behandelt, dass alle unmethylierten Cytosinbasen in Uracil überführt werden, während die 5-Methylcytosinbasen unverändert bleiben;
- die chemisch behandelte DNA-Probe wird amplifiziert unter Verwendung von
- wenigstens zwei Primer-Oligonukleotiden sowie
- einer Polymerase und
- einer Nukleotid-Mischung, enthaltend
entweder
- 2'-Desoxyguanosintriphosphat (dGTP) und
- 2'-Desoxyadenosintriphosphat (dATP) und
- 2'-Desoxythymidintriphosphat (dTTP) oder 2'-Desoxyuridintriphosphat (dUTP),
- und 2'-Desoxycytidintriphosphat (dCTP), mit einer Anfangskonzentration, die höchstens halb so groß ist wie die mittlere Anfangskonzentration der anderen drei Nukleotide für die Amplifikation;
oder
- 2'-Desoxycytidintriphosphat (dCTP) und
- 2'-Desoxyadenosintriphosphat (dATP) und
- 2'-Desoxythymidintriphosphat (dTTP) oder 2'-Desoxyuridintriphosphat (dUTP)
- und 2'-Desoxyguanosintriphosphat (dGTP), mit einer Anfangskonzentration, die höchstens halb so groß ist wie die mittlere Anfangskonzentration der anderen drei Nukleotide für die Amplifikation; und
- auf den Methylierungszustand in der Target-DNA wird aus der Anwesenheit oder Menge eines Amplifikats geschlossen.

2. Verfahren gemäß Anspruch 1, weiterhin **dadurch gekennzeichnet, dass** zusätzlich terminierende Didesoxynukleotide bei der Amplifikation verwendet werden.

3. Verfahren gemäß einem der vorangehenden Ansprüche, weiterhin **dadurch gekennzeichnet, dass** die Denaturierungstemperatur bei der PCR-Amplifikation unterhalb von 90°C liegt.

4. Verfahren gemäß einem der vorangehenden Ansprüche, weiterhin **dadurch gekennzeichnet, dass** die Proben-DNA aus Serum, Plasma, Urin, Sputum oder anderen Körperflüssigkeiten eines Individuums erhalten wird.

5. Verfahren gemäß einem der vorangehenden Ansprüche, weiterhin **dadurch gekennzeichnet, dass** die chemische Behandlung mit einer Bisulfit-, Disulfit- oder Hydrogensulfit-enthaltenden Lösung durchgeführt wird.

6. Verfahren gemäß Anspruch 5, weiterhin **dadurch gekennzeichnet, dass** die chemische Behandlung nach Einbetten der DNA in Agarose durchgeführt wird.

7. Verfahren gemäß Anspruch 5, weiterhin **dadurch gekennzeichnet, dass** bei der chemischen Behandlung ein die DNA-Duplex denaturierendes Reagenz und/oder ein Radikalfänger vorliegt.

8. Verfahren gemäß einem der vorangehenden Ansprüche, weiterhin **dadurch gekennzeichnet, dass** die Amplifikation in Anwesenheit wenigstens eines anderen Oligonukleotids oder eines PNA-Oligomers durchgeführt wird, welches an ein 5'-CG-3'-Dinukleotid oder ein 5'-tG-3'-Dinukleotid oder ein 5'-Ca-3'-Dinukleotid bindet, wodurch das andere Oligonukleotid oder PNA-Oligomer bevorzugt an die Hintergrund-DNA bindet und nachteilig deren Amplifikation beeinflusst und worin "t" ein Thymin in einer Position repräsentiert, welche mit einem unmethylierten Cytosin vor der Bisulfitbehandlung korreliert und "a" mit einer solchen Thymin-Position korreliert.

9. Verfahren gemäß Anspruch 8, weiterhin **dadurch gekennzeichnet, dass** diese Bindungsstelle des anderen Oligonukleotids oder PNA-Oligomers mit den Bindungsstellen der Primer an der Hintergrund-DNA überlappt und das genannte andere Oligonukleotid oder PNA-Oligomer folglich die Bindung wenigstens eines Primer-Oligonukleotids an die Hintergrund-DNA behindert.

10. Verfahren gemäß einem der Ansprüche 8 oder 9, weiterhin **dadurch gekennzeichnet, dass** wenigstens zwei weitere Oligonukleotide oder PNA-Oligomere verwendet werden, wodurch jeweils wieder ihre Bindungsstellen mit der Bindungsstelle eines Primers an der Hintergrund-DNA überlappen und die genannten anderen Oligonukleotide und/oder PNA-Oligomere folglich die Bindung beider Primer-Oligonukleotide an die Hintergrund-DNA behindern.

11. verfahren gemäß einem der Ansprüche 8 bis 10, weiterhin **dadurch gekennzeichnet, dass** diese weiteren Oligonukleotide und/oder PNA-Oligomere in einer Konzentration vorliegen, die fünfmal so groß ist wie die der Primer-Oligonukleotide.

12. Verfahren gemäß einem der vorangehenden Ansprüche, weiterhin **dadurch gekennzeichnet, dass** die verwendete Polymerase keine 5'-3'-Exonucleaseaktivität besitzt.

13. Verfahren gemäß einem der vorangehenden Ansprüche, weiterhin **dadurch gekennzeichnet, dass** die weiteren Oligonukleotide am 5'-Ende modifiziert sind und folglich mittels einer Polymerase mit 5'-3'-Exonucleaseaktivität nicht signifikant abgebaut werden können.

14. Verfahren gemäß einem der vorangehenden Ansprüche, weiterhin **dadurch gekennzeichnet, dass** die Primer in der Amplifikation zwischen Target-DNA und Hintergrund-DNA unterscheiden.

15. Verfahren gemäß Anspruch 14, weiterhin **dadurch gekennzeichnet, dass** jeweils an Positionen an welche wenigstens ein Primer für die Amplifikation bindet, die Hintergrund-DNA methyliert ist, während die Target-DNA unmethyliert ist, wodurch der eine Primer oder die mehreren Primer nach der chemischen Behandlung bevorzugt an die Target-DNA binden.

16. Verfahren gemäß einem der vorangehenden Ansprüche, weiterhin **dadurch gekennzeichnet, dass** zusätzlich wenigstens ein Reporter-Oligonukleotid bei der Amplifikation verwendet wird, dessen Fluoreszenzeigenschaften sich als Folge der Amplifikation verändern.

17. Verfahren gemäß Anspruch 14, weiterhin **dadurch gekennzeichnet, dass** ein Taqman-Assay oder ein LightCycler-Assay oder ein Assay unter Verwendung von Molecular Beacons durchgeführt wird, um im letzten Schritt des Verfahrens auf den Methylierungszustand zu schließen.

18. Verfahren gemäß einem der Ansprüche 16 oder 17, weiterhin **dadurch gekennzeichnet, dass** das Reporter-Oligonukleotid wenigstens einen Fluoreszenz-Marker trägt.

19. Verfahren gemäß einem der Ansprüche 11 bis 15, weiterhin **dadurch gekennzeichnet, dass** das Reporter-Oligonukleotid oder die Reporter-Oligonukleotide die Amplifikation entweder durch eine Erhöhung oder eine Verringerung der Fluoreszenz anzeigt oder anzeigen.

20. Verfahren gemäß Anspruch 19, weiterhin **dadurch gekennzeichnet, dass** die Erhöhung oder Verringerung der Fluoreszenz direkt für die Analyse verwendet wird und ein Rückschluss auf den Methylierungszustand der zu analysierenden DNA aus dem Fluoreszenzsignal gezogen wird.

21. Verfahren gemäß einem der vorangehenden Ansprüche, weiterhin **dadurch gekennzeichnet, dass** aus dem Methylierungsgrad der verschiedenen untersuchten CpG-Positionen ein Rückschluss auf die Anwesenheit einer Krankheit oder eines anderen medizinischen Zustands des Patienten gezogen wird.

22. Verfahren gemäß einem der vorangehenden Ansprüche, weiterhin **dadurch gekennzeichnet, dass** die Amplifikate selbst einen detektierbaren Marker für die Detektion tragen.

23. Verfahren gemäß Anspruch 22, weiterhin **dadurch gekennzeichnet, dass** die Marker Fluoreszenz-Marker sind.

24. Verfahren gemäß Anspruch 22, weiterhin **dadurch gekennzeichnet, dass** die Marker Radionuklide sind.

25. Verfahren gemäß Anspruch 22, weiterhin **dadurch gekennzeichnet, dass** die Marker entfernbare Massenmarker sind, welche in einem Massenspektrometer detektiert werden.

26. Verfahren gemäß einem der vorangehenden Ansprüche, weiterhin **dadurch gekennzeichnet, dass** während der Amplifikation einer der Primer an eine feste Phase gebunden ist.

27. Verfahren gemäß einem der Ansprüche 1 bis 25, weiterhin **dadurch gekennzeichnet, dass** alle Amplifikate im Massenspektrometer detektiert werden und somit eindeutig mittels ihrer Masse charakterisiert werden.

28. Kit, bestehend aus einem Reagenz, enthaltend:
- ein Bisulfit,
- Primer für die Amplifikation und
- eine Nukleotid-Mischung, enthaltend
entweder
- 2'-Desoxyguanosintriphosphat (dGTP) und
- 2'-Desoxyadenosintriphosphat (dATP) und
- 2'-Desoxythymindintriphosphat (dTTP) oder 2'-Desoxyuridintriphosphat (dUTP),
- und 2'-Desoxycytidintriphosphat (dCTP), mit einer Anfangskonzentration, welche höchstens halb so groß ist wie die mittlere Anfangskonzentration der anderen drei Nukleotide für die Amplifikation;
oder
- 2'-Desoxycytidintriphosphat (dCTP) und
- 2'-Desoxyadenosintripohsphat (dATP) und
- 2'-Desoxythymidintriphosphat (dTTP) oder 2'-Desoxyuridintriphosphat (dUTP),
- und 2'-Desoxyguanosintriphosphat (dGTP), mit einer Anfangskonzentration, welche höchstens halb so groß ist wie die mittlere Anfangskonzentration der anderen drei Nukleotide für die Amplifikation.

## Revendications

1. Procédé pour la détection d'une méthylation de cytosine dans des échantillons d'ADN, **caractérisé en ce que** les étapes suivantes sont menées :
- un échantillon d'ADN génomique, qui comprend de l'ADN à étudier (ADN cible) et de l'ADN de second plan, est traité par voie chimique de sorte que toutes les bases cytosine non méthylées sont converties en uracile, tandis que les bases 5-méthylcytosine restent inchangées ;
- l'échantillon d'ADN traité par voie chimique est amplifié par l'utilisation de
- au moins deux oligonucléotides amorces, ainsi que
- une polymérase, et
- un mélange de nucléotides contenant
soit
- du 2'-désoxyguanosine triphosphate (dGTP), et
- du 2'-désoxyadénosine triphosphate (dATP), et
- du 2'-désoxythymidine triphosphate (dTTP) ou du 2'-désoxyuridine triphosphate (dUTP),
- et du 2'-désoxycytidine triphosphate (dCTP), à une concentration initiale qui est au plus la moitié de la concentration initiale moyenne des trois autres nucléotides pour l'amplification ;
soit
- du 2'-désoxycytidine triphosphate (dCTP), et
- du 2'-désoxyadénosine triphosphate (dATP), et
- du 2'-désoxythymidine triphosphate (dTTP) ou du 2'-désoxyuridine triphosphate (dUTP)
- et du 2'-désoxyguanosine triphosphate (dGTP), à une concentration initiale qui est au plus la moitié de la concentration initiale moyenne des trois autres nucléotides pour l'amplification ; et
- l'état de méthylation dans l'ADN cible est conclu à partir de la présence ou la quantité d'un amplificat.

2. Procédé selon la revendication 1, **caractérisé en outre en ce que** des didésoxynucléotides de terminaison sont en plus utilisés dans l'amplification.

3. Procédé selon l'une des revendications précédentes, **caractérisé en outre en ce que** la température de dénaturation reste inférieure à 90°C dans l'amplification par PCR.

4. Procédé selon l'une des revendications précédentes, **caractérisé en outre en ce que** l'ADN échantillon est obtenu à partir de sérum, plasma, urine, salive ou d'autres fluides corporels d'un sujet.

5. Procédé selon l'une des revendications précédentes, **caractérisé en outre en ce que** le traitement chimique est mené avec une solution contenant un bisulfite, un disulfite ou un sulfite d'hydrogène.

6. Procédé selon la revendication 5, **caractérisé en outre en ce que** le traitement chimique est mené après inclusion de l'ADN dans de l'agarose.

7. Procédé selon la revendication 5, **caractérisé en outre en ce que** dans le traitement chimique, un réactif qui dénature l'ADN duplex et/ou un antiradicaux est présent.

8. Procédé selon l'une des revendications précédentes, **caractérisé en outre en ce que** l'amplification est menée en présence d'au moins un autre oligonucléotide ou un oligomère PNA, qui se lie à un dinucléotide 5'-CG-3' ou un dinucléotide 5'-tG-3' ou un dinucléotide 5'-Ca-3', moyennant quoi l'autre oligonucléotide ou oligomère PNA se lie de préférence à l'ADN de second plan et affecte négativement son amplification et dans lequel « t » représente une thymine à une position qui se corrèle avec une cytosine non méthylée avant un traitement au bisulfite et « a » se corrèle avec une telle position de thymine.

9. Procédé selon la revendication 8, **caractérisé en outre en ce que** ce site de liaison de l'autre oligonucléotide ou oligomère PNA chevauche les sites de liaison des amorces sur l'ADN de second plan, et ledit autre oligonucléotide ou oligomère PNA empêche ainsi la liaison d'au moins un oligonucléotide amorce à l'ADN de second plan.

10. Procédé selon l'une des revendications 8 ou 9, **caractérisé en outre en ce qu'**au moins deux autres oligonucléotides ou oligomères PNA sont utilisés, moyennant quoi leurs sites de liaison chevauchent chacun de nouveau le site de liaison d'une amorce à l'ADN de second plan et lesdits autres oligonucléotides et/ou oligomères PNA gênent ainsi la liaison des deux oligonucléotides amorces à l'ADN de second plan.

11. Procédé selon l'une des revendications 8 à 10, **caractérisé en outre en ce que** ces autres oligonucléotides et/ou oligomères PNA sont présents à au moins cinq fois la concentration des oligonucléotides amorces.

12. Procédé selon l'une des revendications précédentes, **caractérisé en outre en ce que** la polymérase utilisée ne possède aucune activité exonucléase 5'-3'.

13. Procédé selon l'une des revendications précédentes, **caractérisé en outre en ce que** les autres oligonucléotides sont modifiés au niveau de l'extrémité 5' et ainsi ne peuvent être significativement dégradés par une polymérase avec une activité exonucléase 5'-3'.

14. Procédé selon l'une des revendications précédentes, **caractérisé en outre en ce que** les amorces dans l'amplification font la distinction entre l'ADN cible et l'ADN de second plan.

15. Procédé selon la revendication 14, **caractérisé en outre en ce que** l'ADN de second plan est méthylé, tandis que l'ADN cible est non méthylé, chacun à des positions auxquelles au moins une amorce pour l'amplification se lie, moyennant quoi l'une des amorces ou plus se lie(lient) de préférence à l'ADN cible après le traitement chimique.

16. Procédé selon l'une des revendications précédentes, **caractérisé en outre en ce que** en plus au moins un oligonucléotide rapporteur est utilisé dans l'amplification, dont les propriétés de fluorescence changent à la suite de l'amplification.

17. Procédé selon la revendication 14, **caractérisé en outre en ce qu'**un essai Taqman ou un essai LightCycler ou un essai avec l'utilisation de balises moléculaires est mené pour conclure sur l'état de méthylation à la dernière étape du procédé.

18. Procédé selon l'une des revendications 16 ou 17, **caractérisé en outre en ce que** l'oligonucléotide rapporteur porte au moins une marque fluorescente.

19. Procédé selon l'une des revendications 11 à 15, **caractérisé en outre en ce que** l'oligonucléotide rapporteur ou les oligonucléotides rapporteurs indique ou indiquent l'amplification soit par une augmentation soit par une diminution de la fluorescence.

20. Procédé selon la revendication 19, **caractérisé en outre en ce que** l'augmentation ou la diminution de fluorescence est utilisée directement pour l'analyse et une conclusion sur l'état de méthylation de l'ADN à analyser est réalisée à partir du signal fluorescent.

21. Procédé selon l'une des revendications précédentes, **caractérisé en outre en ce qu'**une conclusion est réalisée sur la présence d'une maladie ou d'un autre état pathologique du patient à partir du degré de méthylation des différentes positions CpG étudiées.

22. Procédé selon l'une des revendications précédentes, **caractérisé en outre en ce que** les amplificats eux-mêmes portent une marque détectable pour la détection.

23. Procédé selon la revendication 22, **caractérisé en outre en ce que** les marques sont des marques fluorescentes.

24. Procédé selon la revendication 22, **caractérisé en outre en ce que** les marques sont des radionucléides.

25. Procédé selon la revendication 22, **caractérisé en outre en ce que** les marques sont des marques de masse détachables qui sont détectées dans un spectromètre de masse.

26. Procédé selon l'une des revendications précédentes, **caractérisé en outre en ce que** pendant l'amplification, une des amorces est liée à une phase solide.

27. Procédé selon l'une des revendications 1 à 25, **caractérisé en outre en ce que** tous les amplificats sont détectés dans le spectromètre de masse et sont ainsi clairement **caractérisés par** leur masse.

28. Kit constitué d'un réactif contenant :
- un bisulfite,
- des amorces pour l'amplification, et
- un mélange de nucléotides, contenant
soit
- du 2'-désoxyguanosine triphosphate (dGTP), et
- du 2'-désoxyadénosine triphosphate (dATP), et
- du 2'-désoxythymidine triphosphate (dTTP) ou du 2'-désoxyuridine triphosphate (dUTP),
- et du 2'-désoxycytidine triphosphate (dCTP), à une concentration initiale qui est au plus la moitié de la concentration initiale moyenne des trois autres nucléotides pour l'amplification ;
soit
- du 2'-désoxycytidine triphosphate (dCTP), et
- du 2'-désoxyadénosine triphosphate (dATP), et
- du 2'-désoxythymidine triphosphate (dTTP) ou du 2'-désoxyuridine triphosphate (dUTP),
- et du 2'-désoxyguanosine triphosphate (dGTP), à une concentration initiale qui est au plus la moitié de la concentration initiale moyenne des trois autres nucléotides pour l'amplification.
